# EUROPEAN PATENT APPLICATION

(11) **EP 1 732 021 A1**
(43) Date of publication of application: **13.12.2006**
(21) Application number: 05721340.7
(22) Date of filing: 23.03.2005
(51) Int. Cl.: G06F 19/00, C12M 1/00, C12N 15/09, C12Q 1/68, G06F 17/30

(54) **METHOD OF SEARCHING SPECIFIC BASE SEQUENCE**

(30) Priority: 26.03.2004 JP 2004093301
(71) Applicant: Bio-Think Tank Co., Ltd., Tokyo 113-0033 (JP)
(72) Inventor: MORISHITA, Shinichi, 1790073 (JP); YAMADA, Tomoyuki, Bunkyo-ku, Tokyo 1130033 (JP); NAITO, Yuki, 1020082 (JP)
(74) Representative: Betten & Resch
(86) International application number: PCT/JP2005/005290
(87) International publication number: WO 2005/093631

(57) **Abstract**

It is intended to efficiently determine a base sequence specifically appearing in an expression gene. For this, providing that the expression gene consists of exons (301)...(306) and especially that exon (301) is united with exon (302) and exon (302) with exon (303), an aggregate of base sequences (401) (403) being a union of exon base sequences (301)...(305) and a boundary base sequence obtained by uniting together base sequences(404) and (405) and base sequences (406) and (407) respectively existing over boundaries between exon (301) and exon (302) and between exon (302) and exon (303) is formed, and the aggregate is searched. If a base sequence is one specifically appearing in the expression gene, the number of search results is 1 and otherwise, the number is plural.

## Description

### Field of the Invention

The present invention relates to a method, an apparatus, and a program used to search for a specific base sequence appearing in a genetic base sequence.

### Description of the Related Art

The study on gene information related to a base sequence was developed according to the elucidation of the DNA (Deoxyribonucleic Acid) structure by Watson and Crick. The structure of DNA is made up of a nucleotide sequence including any one of the bases of adenine (A), cytosine (C), guanine (G), or thymine (T), and the double-helix structure, in which, normally, base pairs of A and T, and G and C are formed in the nucleus of a cell.

It is known that the nucleotide sequence of DNA expressing a gene (hereinafter, referred to as 'gene sequence') is transcribed to RNA (Ribonucleic Acid), and spliced, thereby generating mRNA (messenger RNA), and synthesizing protein. RNA is a nucleic acid having D-ribose as a sugar component, and adenine (A), cytosine (C), guanine (G), or uracil (U) as a base. In the gene sequence, portions having protein information are called exons, and the others are called introns. Accordingly, introns of RNA are removed by splicing.

In recent years, the phenomenon called RNA interference was discovered. The RNA interference is a phenomenon in which the double-stranded RNA of a cell breaks mRNA having a specific sequence, thereby suppressing gene expression. This phenomenon is found in the experiment using nematode cell at the outset. Subsequently, it was discovered that this phenomenon exists in mammal cells, and the phenomenon was focused upon. The reason for this is that, by causing RNA interference artificially, the action of a specific gene is suppressed, so that it becomes possible to study the action of a specific gene. In addition, as a result of the discovery of RNA interference, it has become possible to develop medicine that suppresses the action of a specific gene.

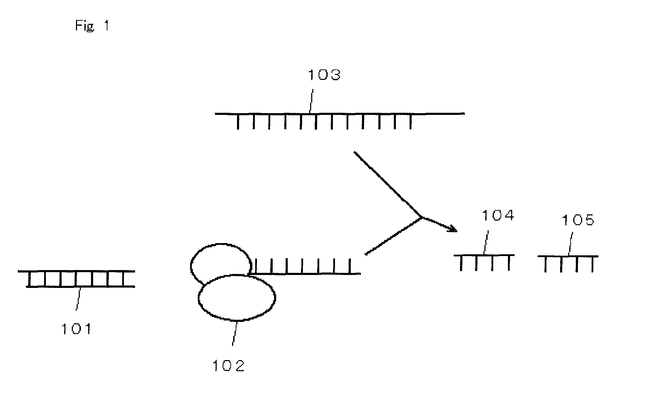

Fig. 1 is a schematic diagram showing the process of RNA interference. RNA interference occurs in the following process. ₛᵢRNA (short interfering RNA) 101, having a length of about 21 to 23 base pairs, is concatenated to multi-complex proteins, thereby forming RISC (RNA-induced silencing complex) 102. RISC is concatenated to mRNA 103, which shares homology with the ₛᵢRNA, thereby breaking the mRNA, so that the mRNA becomes dysfunctional (in Fig. 1, fragments 104 and 105 are fragments of broken mRNA). Here, the term 'two base sequences share homology' means that two base sequences have complementarity, or imperfect complementarity. Here, 'complementarity' means that in two entire base sequences, a pair of A and T, G and C, and A and U are perfectly formed. Accordingly, the term homology means that, in a portion of two base sequences, a pair, other than the three types of pairs A and T, G and C, and A and U, which are base pairs having complemnetarity, is formed. Note that, as described hereinbelow, it is determined whether the two base pairs share homology based on how many base pairs having complementerity between two base sequences exist in what case. Therefore, in RNA interference, there are some cases, in which complementerity of more than 80%, preferably 90%, and more preferably 95%, appears, it is determined that the two base pairs share homology. Moreover, not only the percentage of base pair having complementarity, but also the number of series of bases appearing successively in the base sequence, is considered; the existence of homology between two base sequences is determined in some cases. Furthermore, it is known that there is a possibility of G and U forming a pair, in addition to the three types of pairs of A and T, G and C, and A and U, which are base pairs having complementarity, so that, considering the existence of the pair of G and U, there is a possibility of a determination of the existence of homology.

Accordingly, in order to cause RNA interference, and to suppress the action of the targeted gene, it is important to determine the sequence of ₛᵢRNA. Therefore, it is important to determine the sequence of ₛᵢRNA, which appears only in the target gene and does not share homology with the base sequence of the other gene.

Note that, in the case of mammals, it is known that not all ₛᵢRNA, which share the homology with a specific area of a certain gene, cause RNA interference. For this reason, the method for evaluating a base sequence of ₛᵢRNA for causing RNA interference has been suggested (e.g. Non-patent document 1). As seen from this finding, the present invention may be carried out in the preliminary stage of the evaluation of the base sequence. Alternatively, after the evaluation of the base sequence, the present invention may be carried out, so that the base sequence, sharing homology with a specific area, is acquired from the highly valued base sequence.

Moreover, in recent years, gene analysis or gene examination using a microarray has been carried out. The 'microarray' is a kind of DNA chip, in which oligo-DNA, having a length of 15 to 30 base pairs, is synthesized on a glass plate etc. (e.g. Non-patent document 2)

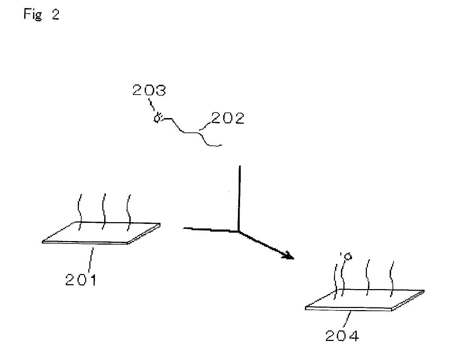

Fig. 2 is a diagram exemplifying processes of gene analysis or of gene examination etc. using microarray. When flowing DNA (202), which is fluorochrome-labeled with the label 203, on the microarray 202, in which oligo-DNA is synthesized on a glass plate etc., the oligo-DNA on the microarray sharing complementarity or homology is hybridized with the DNA (portion 204). By detecting fluorescence with the fluorescence dye of the label, it is determined at what position the DNA is hybridized with oligo-DNA, thereby determining the type of DNA (202). Although only several oligo-DNA are indicated on the microarray in Fig. 2, literally, tens of thousands of oligo-DNA exist in the 0.5 square inch area of a microarray.

Therefore, in designing a microarray, it is quite important to determine the base sequence of the oligo-DNA to be arranged on a microarray.

Non-patent document 1: 'Rational siRNA design for RNA interference', Angela Reynold et al., Nature Biotechnology, Published online 1 February 2004.

Non-patent document 2: 'Genetic chemistry', Naoki Sugimoto, Kagaku-Dojin Publishing Company, Inc., 2002.

It is an objective of the present invention to implement an effective determination of a specific base sequence appearing in a specified gene. The term 'specific' means that the base sequence appears only in the targeted gene and does not appear in another gene. Thus, the base sequence of ₛᵢRNA, used to repress only the specific gene, is acquired. In addition, the sequence of oligo-DNA, used to detect only the specific gene, is acquired.

Although a database of the base sequence of a gene has already been constructed, it has deficiencies in determining the specific base sequence. The above deficiencies will be described hereinbelow.

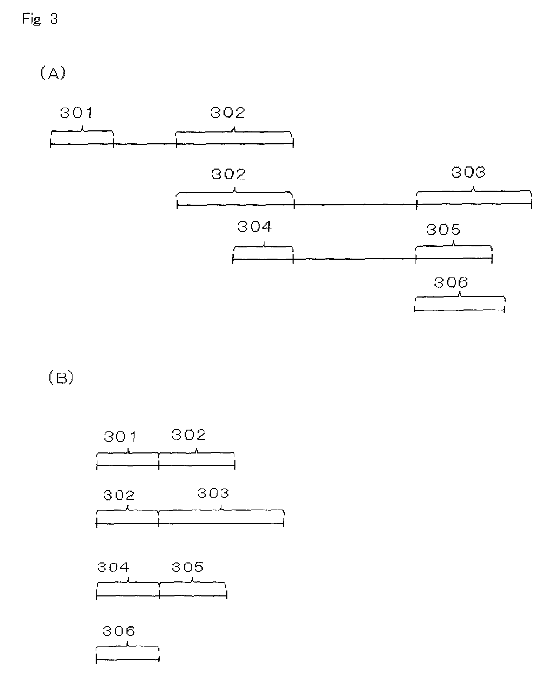

Fig. 3 shows the relationship between the DNA sequence and the expressed gene sequence transcribed to mRNA. Fig. 3 (A) shows portions of four DNA sequences. In Fig. 3 (A), one portion of the one DNA sequence is indicated in an easy-to-understand manner, and the base sequences of the same portion are indicated so that there is a corresponding relationship between the upper and the lower sequences. It is known that, in a DNA sequence, there are exons forming an expressed gene and introns not forming an expressed gene. In Fig. 3 (A), 301, 302, 303, 304, 305, and 306 are exons, and the others are introns. Fig. 3 (B) shows expressed gene sequences. As shown in Fig. 3 (B), one exon does not always appear in only one expressed gene sequence, and can appear in a plurality of expressed gene sequences. For example, the exon 302 is concatenated to the exon 301, thereby forming an expressed gene, and is concatenated to the exon 303, thereby forming the other expressed gene.

In addition, the case, in which a portion of an exon is the exon, may exist. For example, in Fig. 3 (A), a portion of the exon 302 is the exon 304, and portions of the exon 303 are the exons 305 and 306.

Therefore, in a database storing expressed gene sequences, the base sequence of one exon, or a portion thereof, appears in a plurality of expressed genes. Therefore, for example, if a search of the specific base sequence appears in the exon 302 is carried out, some base sequences can be detected, so that it is possible to determine that the base sequence is not a specific base sequence. In order to exclude the possibility, if multiple base sequences are detected, it is necessary to examine the search result, and to separately check whether the sequence is a specific sequence appearing only in a specific exon.

In order to avoid the above case, there is a method for carrying out a search on the entire genome sequence. However, in this search, the base sequence, which straddles exon borders of expressed gene sequences, is not detected. Therefore, cases in which the expressed gene sequence is formed by concatenating multiple exons in the genome sequence, and a portion of the base sequence is included in an exon, and the other portions of the base sequence are included in the other exon, the exon border, which is a base located on the end of the exon, is included in the base sequence; the base sequence does not appear in the genome sequence, so that it is not detected. For this reason, if a base sequence, which straddles exon borders of an expressed gene sequence, is detected multiple times, it is impossible to determine that the base sequence is not a specific base sequence, or to determine that the sequence is specific even if the sequence, which straddles exon borders, is specific.

### SUMMARY OF THE INVENTION

It is an objective of the present invention to provide a method, an apparatus, a database, and a program for effective detection of a specific base sequence appearing in an expressed gene, more specifically, a specific base sequence appearing in one exon, or specific base sequence appearing in expressed gene by exon concatenating.

In the present invention, a search is carried out using a union of sets of a union of sets of exon base sequences, and a set of border base sequences, which straddle exon borders in the expressed gene formed by a plurality of exons. Consequently, if the base sequence appearing in expressed gene sequence is specific, the number of search results is one, and if not, the number of search results is multiple. As a result, by examining the search result, it is possible to immediately determine whether the base sequence is the specific base sequence, so that the above deficiencies are overcome.

In addition, the base sequence, which straddles exon borders in the expressed gene, may be appropriately integrated, so that it becomes possible to reduce the number database records.

Additionally, in order to specify a homological level, the number of allowable mismatching bases in the search, may be specified. In addition, in order to specify the homological level, mismatching base pairs may be specified, or distribution of occurrences of mismatches may be specified. An example of the specified distribution includes length of successive bases, which are not determined to be mismatching (therefore, the length in which base pairs appear successively). If this length exceeds a certain length, in RNA interference, even if a mismatching base sequence exists, ₛᵢRNA is concatenated to mRNA. In order to exclude the biding, the length of successive non-mismatching base pairs is specified.

Moreover, in the present invention, information as to which portion of the genome sequence is exon or intron greatly affects the configuration of the database of base sequence used in the search. Although, in the description below, it is assumed that the result, which has been studied, is used, the future study result may be used for configuring the database of the base sequence.

According to the present invention, it becomes possible to determine whether a base sequence is a specific base sequence appearing in expressed gene on the basis of the number of search results by generating a set of base sequences from exon base sequences and base sequences appearing at exon borders, and by carrying out the search.

### BRIEF DESCRIPTION OF THE DRAWINGS

Fig. 1 is a schematic diagram showing the process ofRNA interference;
Fig. 2 is a diagram exemplifying processes of gene analysis or of gene examination etc. using microarray;
Fig. 3 is a diagram exemplifying a relationship between a DNA sequence and an expressed gene sequence transcribed to mRNA;
Fig. 4 is a diagram exemplifying a union of sets of exons and a base sequence straddling exon borders of expressed genes;
Fig. 5 is a diagram exemplifying N-1 border base sequences;
Fig. 6 is a diagram explaining integration of base sequences;
Fig. 7 is a diagram explaining integration of base sequences;
Fig. 8 is a table used for computation of a union of sets of base sequences;
Fig. 9 is a flow chart used for computation of a union of sets of base sequences;
Fig. 10 is a diagram exemplifying computation of an integration of border base sequences;
Fig. 11 is a diagram exemplifying the case where an exon, of which length is less than N-1mer, exists;
Fig. 12 is a table used for operation of integration;
Fig. 13 is a flow chart of the integration process;
Fig. 14 is a flow chart of the process of the generation method for set of base sequences of the first embodiment of the present invention;
Fig. 15 is a table storing the base sequence acquired by the generation step for union of sets;
Fig. 16 is a flow chart of the method for searching for specific base sequences of the second embodiment of the present invention;
Fig. 17 is a flow chart of the method for searching for specific base sequences of the fourth embodiment of the present invention;
Fig. 18 is a diagram showing a mismatch between base sequences, which cannot be detected by BLAST in the case that the length of base sequence candidate is 19 and the allowable number of matches is 3;
Fig. 19 is a functional block diagram of the apparatus for searching for specific base sequences of the ninth embodiment of the present invention;
Fig. 20 is a functional block diagram of the apparatus for searching for specific base sequences of the eleventh embodiment of the present invention;
Fig. 21 is a functional block diagram of the apparatus for searching for specific base sequences of the twelfth embodiment of the present invention;
Fig. 22 is a functional block diagram of the apparatus for searching for specific base sequences of the thirteenth embodiment of the present invention.

### DETAILED DESCRIPTION OF THE PREFERRED EMBODIMENTS

Embodiments of the present invention will be described hereinbelow with reference to the drawings. The present invention is not to be limited to the above embodiments and able to be embodied in various forms without departing from the scope thereof.

Before the description of the embodiments, the outline of the present invention will be described in some sections.

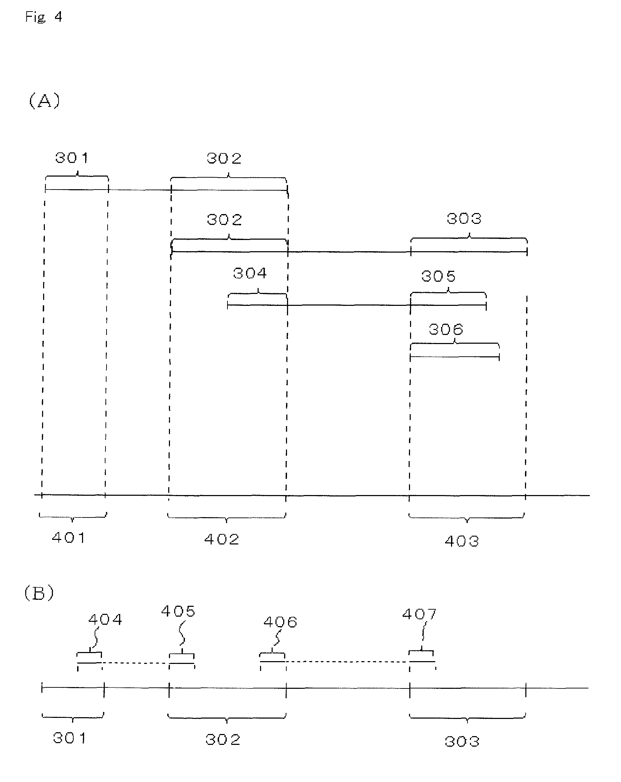

Fig. 4 is a diagram exemplifying a union of sets of exons and base sequences straddling exon borders of expressed genes. Note that, hereinbelow, the base sequence straddling exon borders of expressed genes is referred to as 'border base sequence'.

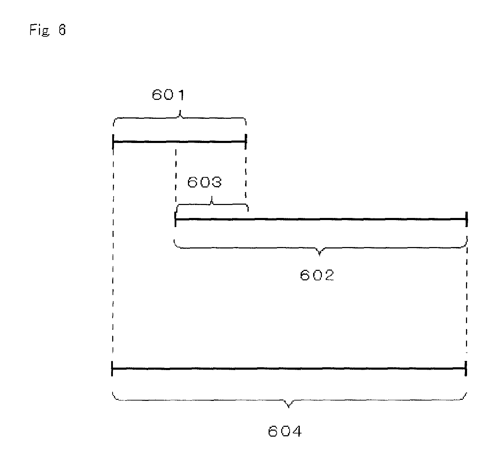

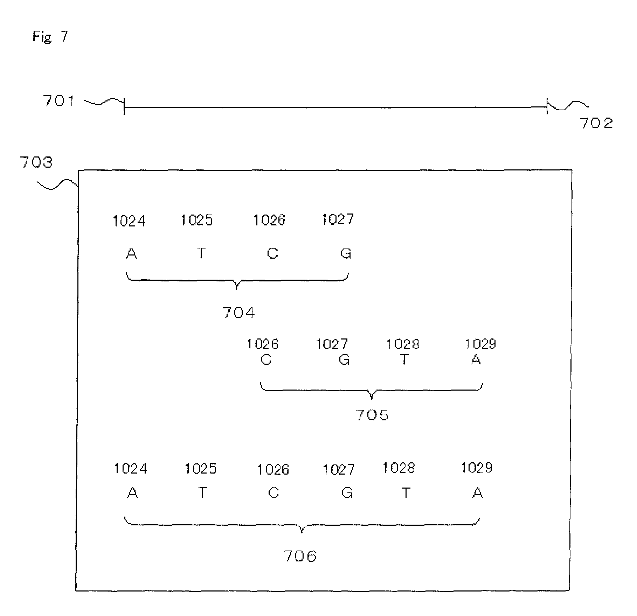

Fig. 4 (A) is a diagram explaining a union of sets of exon base sequences. As with Fig. 3 (A), Fig. 4 (A) shows portions of four DNA sequences. In Fig. 4 (A), one portion of one DNA sequence is indicated, and the base sequences of the same portion are indicated so that there is a corresponding relationship between the upper and the lower sequences. The relationship of exons 301, 302, 303, 304, 305, and 306 is as shown in Fig. 4 (A). Therefore, there is no exon, which overlaps or has an inclusive relation with exon 301, exon 304 is a portion of exon 302, and exon 305 and exon 306 are portions of exon 303. In this case, the sequence 401, 402, and 403 are acquired as union of sets of these exons. Therefore, sequence 401 is, itself, exon 301, and sequence 402 is a union of exon 302 and exon 304. Since exon 304 is a portion of exon 302, sequence 402 is, itself, exon 302. Similarly, sequence 403 is, itself, exon 303. In Fig. 4, like the relationship between exon 302 and exon 304, the case, in which one exon includes the other exon, is shown. There is another case, not the case of an inclusive relation, where portions of two exon base sequences are overlapping each other. This case will be described with reference to Fig. 6 and 7 etc.

The lower part of Fig. 4 is a diagram explaining a border base sequence. In cases where exon 301 and exon 302 are concatenated, so that an expressed gene is formed; the base sequence, in which the right-side portion 404 and the left-side portion 405 on the border of the concatenating site are concatenated, is the border base sequence. Similarly, in cases where exon 302 and exon 303 are concatenated, so that an expressed gene is formed; the base sequence, in which the right-side portion 406 and the left-side portion 407 on the border of the concatenating site are concatenated, is the border base sequence. Note that the length of the border sequence corresponds to the length of the base sequence, which is for searching whether it specifically appears in expressed gene sequence. Assuming that the length is N, there are N-1 border base sequences.

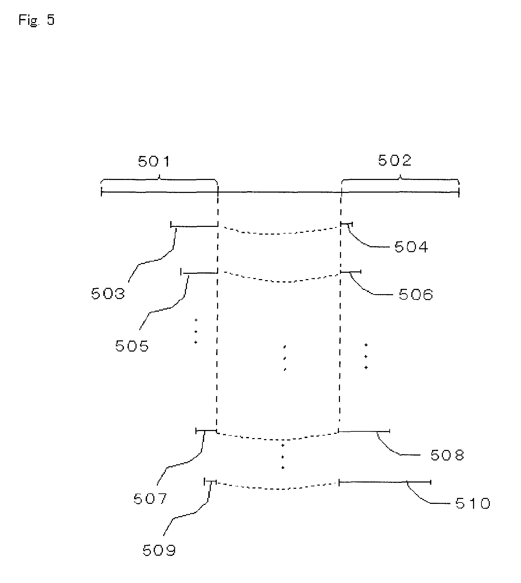

Fig. 5 shows N-1 border base sequences. Assuming that exon 501 and exon 502 are concatenated, thereby forming the expressed gene, portion 503, which is the right end of exon 501 and N-1mer ('mer' is a unit of length of base sequence, and the length of 1 base is 1mer), and portion 504, which is the left end of exon 504 and 1mer, are concatenated, thereby acquiring one border base sequence. Similarly, portion 505, which is N-2mer, and portion 506, which is 2mer, are concatenated, portion 507, which is 2mer, and portion 508, which is N-2mer, are concatenated, and portion 509, which is 1mer, and portion 510, which is N-1mer, are concatenated; thereby acquiring N-2 base sequences. These N-1 base sequences have overlapping relationships in one portion, not relationships of inclusion, so that it is possible to integrate them into one.

Fig. 6 is a diagram explaining the integration of base sequences. Therefore, it is indicated that if base sequence 601 overlaps base sequence 602 in portion 603, base sequence 601 and base sequence 602 are integrated, thereby acquiring base sequence 604. Base sequence 604 is acquired by concatenating three portions, the portion of base sequence 601, except the portion 603, portion 603, and a portion of base sequence 602, except portion 603.

Fig. 7 is a diagram precisely explaining the integration. As shown in the upper portion of Fig. 7, the bases forming base sequence of DNA can be assigned numbers, in order from the end-base of DNA (e.g. the end called as '5' end' in DNA chemical structure), as 1. For example, if the end point 701 is '5' end, and the end point 702 is '3' end, it is possible to assign numbers to the bases as 1,2,3, and so on, from the base of the end point 701. Hereinafter, these numbers are referred to as base position. For example, in the lower portion 703 of Fig. 7, the number 1024 is assigned on the base A appearing in the base sequence 704. This means that the base A is the 1024th base from the '5' end of DNA. The base sequence 704 overlaps with 705 in only one portion. Therefore, the 1026th base sequence overlaps the 1027th in one portion. In this case, by integrating the base sequence 704 and 705, the base sequence 706 is acquired.

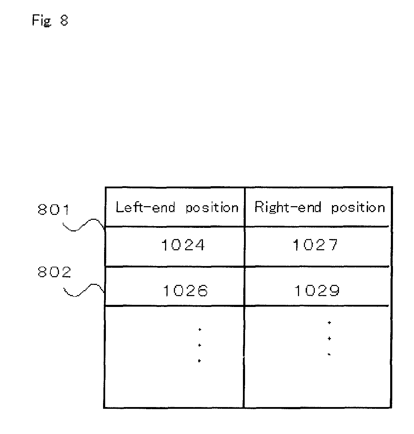

Fig. 8 is a table used for computation of a union of sets, specifically, an integration of base sequences. Here, the 'computation' is preferably carried out by a computer program. In this case, the table may be managed by the database management system etc. The table in Fig. 8 includes columns named 'left-end position' and 'right-end position'. The respective rows store the left-end and right-end base positions of the exon base sequence. In addition, the left-end and right-end base positions of the exon base sequence, which straddle exon borders, may be stored (as described hereinbelow, there are some cases where difficult operations are required for the integration of base sequences, which straddle exon borders, so that the table of Fig. 8 can be used in some limited cases). Note that, respective rows of the table, a row number is assigned, for example, to row 801, the number 1 is assigned, and to row 802, the number 2 is assigned. Accordingly, row 801 is called 'the first row' and row 802 is called 'the second row'.

In addition, the attribute information of the exon, which is correlated with the respective rows stored in the table of Fig. 8, may be stored. For example, there may be another table, which stores the attribute information of the exon correlated with the row number in the table of Fig. 8. Alternatively, the attribute information of the exon may be stored in the column, which is added to the table of Fig. 8. Here, the 'attribute information' corresponds to information including: (1) information indicating sequence position of the exon, or (2) information for identifying the gene formed by the exon. The 'information indicating sequence position of exon' is information indicating in which position of the genome sequence the exon is located. For example, the position from the end of the DNA. Although this information is stored in the column at the left-end position or the right-end position of the table of Fig. 8, since the value stored in the column at the left-end position or the right-end position changes upon computing the union of sets, the information may be stored separately. In addition, the 'information for identifying gene formed by exon' corresponds to information indicating the gene including the exon base sequence such as the name of the gene etc. An example of the information includes the length of exon other than the information indicating the sequence position of exon, and the information for identifying the gene formed by the exon.

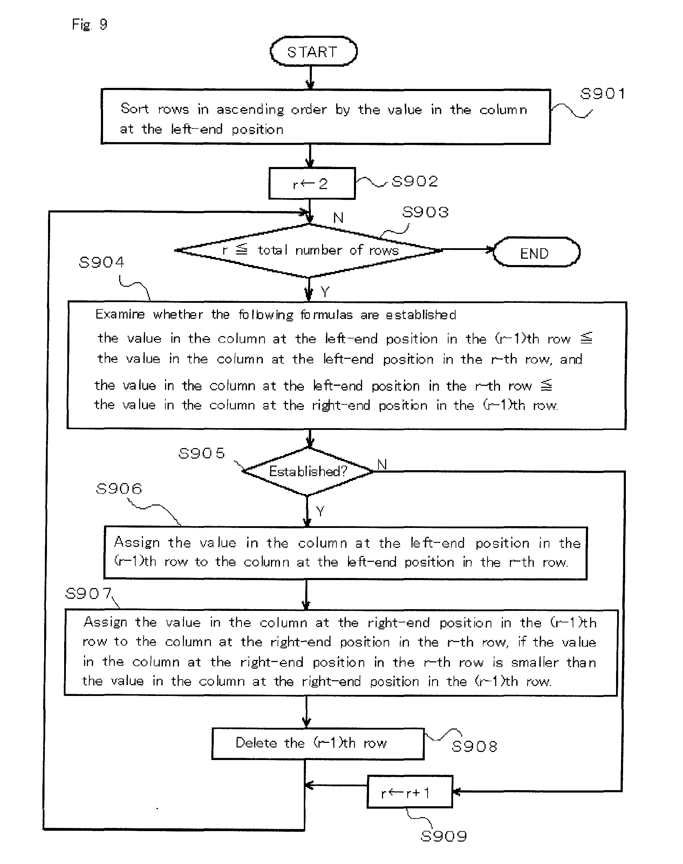

Fig. 9 is a flow chart used for the computation of a union of sets, specifically integration of base sequences. As described above, 'computation' is preferably carried out by a computer program. Accordingly, the processing of the flow chart of Fig. 9 is preferably carried out using a computer. In step S901, rows are sorted in ascending order based on the value in the column named as the left-end position. Therefore, the rows in the table of Fig. 8 are sorted, so that the value in the column, named as the left-end position, in the N+1th row is not less than the value in the column, named as the left-end position, in the Nth row. Subsequently, in step S902, 2 is assigned as a variable 'r'. The variable 'r' is a variable indicating which row is currently being processed.

In step S903, it is determined whether the value of r is less than the value of all rows. Therefore, it is determined whether the r-th row exists in the table. If so, (step S903: in the case of branching to Y), the steps after S904 are carried out. If not, (step S903: in the case of branching to N), the processes of all rows are completed.

In step S904, it is examined whether the base indicated in the r-th row and the base sequence indicated in the (r-1)th row have an inclusive relation or relation of partial overlap. Therefore, it is examined whether the value in the column at the left-end position in the (r-1)th row ≦ the value in the column at the left-end position in the r-th row, and the value in the column at the left-end position in the r-th row ≦ the value in the column at the right-end position in the (r-1)th row. In step S905, if the above formulas are true (step S905: in the case of branching to Y), step S906 is carried out, and if not (step S905: in the case of branching to N), step S909 is carried out.

In step S906, the value in the column at the left-end position in the (r-1)th row is assigned to the column at the left-end position in the r-th row. In step S907, if the value in the column at the right-end position in the r-th row is smaller than the value at the right-end position in the (r-1)th row, the value at the right-end position in the (r-1)th row is assigned to the column at the right-end position in the r-th row. In step S907 and S907, the integration of the base sequences indicated in the (r-1)th row and the r-th row is indicated in the r-th row. Therefore, the (r-1)th row becomes unnecessary, and deleted in step S908. Thus, the value of the total number of rows is reduced by 1. After that, the processing returns to step S903. Note that in step S908, the (r-1)th row may be moved to another table and stored therein, but may not be deleted. This makes it possible, for example, to store information as to which sequence is the base of the position of the exon in the other table, thereby enabling a search.

In addition, in step S907, the attribute information correlated with the r-th row may bemerged with the attribute information correlated with the (r-1)th row. For example, the strings expressing the attribute information correlated with the r-th row are concatenated with the strings expressing the attribute information correlated with the (r-1)th row. The strings acquired by this concatenation may be stored as the attribute information correlated with the (r-1)th row. For example, if 'A' and 'B', correlated with the (r-1)th row, are stored as 'A, B' by using ',' as a separator, and 'C', correlated with the r-th row, is stored; 'A, B, C', which is acquired by concatenating 'A, B' by using ',' with 'C' as a separator, may be correlated with the (r-1)th row and stored. This makes it possible to know which exon is the base of an element of a union of sets of exons, for example, and which gene is related.

In step S909, in order to carry out the process for the subsequent row, the value of r is increased by 1, after that, the processing is back to step S903.

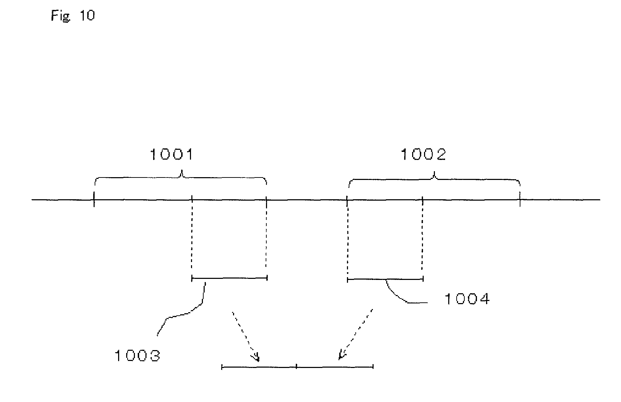

Fig. 10 is a diagram exemplifying the computation of the integration of N-1 border base sequences in the case where two exons are concatenated and form the expressed gene. Assuming that the exon 1001 and 1002 are concatenated and form the expressed gene, in this case, the base sequence, which is an integration of the border base sequences in the border between the exon 1001 and 1002, is 2N-2mer base sequence, in which the N-1mer base sequence 1003, at the right-end of the exon 1001, and the N-1mer base sequence 1004, at the left-end of the exon 1002 are concatenated. Note that, in Fig. 10, the length of the exon 1001 and 1002 are required to be more than N-1 mer, respectively.

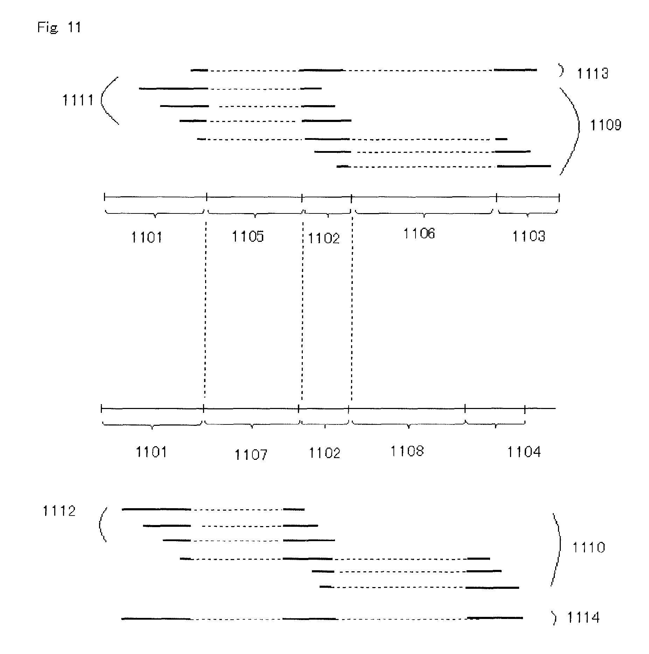

Fig. 11 is a diagram exemplifying the case where an exon, whose length is less than N-1mer, exists. In Fig. 11, the portion 1101, 1102, 1103, and 1104 are exons, and the exon 1101, 1102, and 1103 are concatenated and form the expressed gene, and the exon 1101, 1102, and 1104 are concatenated and form the other expressed gene. In addition, the length of the exon 1102 is less than N-1mer, and the exon 1103 and 1104 has an overlapping relation in one portion. The portion 1105, 1106, 1107, and 1108 are the introns.

In this case, the border base sequence is computed, so that the portions indicated by a solid line in 1109 and 1110 are acquired. The search for determining whether they are the specific base sequences appearing in the expressed gene is carried out on the set, in which the set of these border sequences is added to the union of sets of the exon 1101,1102,1103, and 1104. In addition, instead of the set of these border sequences, the set of base sequences acquired by the operations of integration to the set of border base sequences, which will be described hereinbelow, may be used.

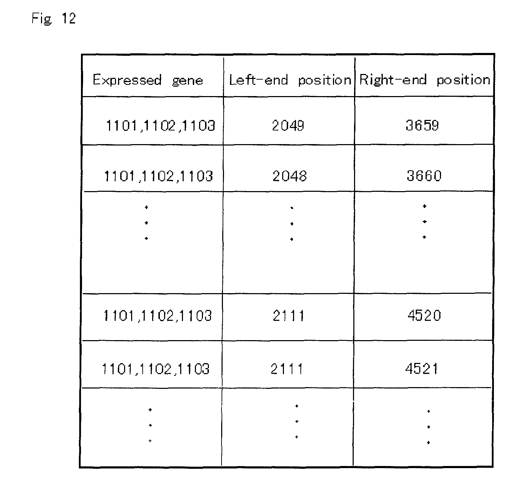

Fig. 12 is a table used for operation of integration. The table consists of the column of 'expressed gene', 'left-end position', and 'right-end position'. The column of 'expressed gene' stores the identifier for identifying the expressed gene in which the border base sequence appears. In Fig. 12, such identifiers are indicated by arranging the codes of exons forming the expressed gene. The 'left-end position' and the 'right-end position' correspond to those in the table of Fig. 8, and store the positions of the left-end base and the right-end base of the border base sequence. Note that the operation of integration can be carried out by computer. In this case, the table may be managed by a database management system, and may be processed. In addition, the above program may be recorded on a medium such as a flexible disk, an optical disk, or a memory stick.

First, one of the rows in the table of Fig. 12 is generated corresponding to one border base sequence. The unique combination of the values in the columns of the 'left-end position' and the 'right-end position' is generated, so that the set of border base sequences is stored in the table. Therefore, the processing is carried out, so that the combination of the values in the columns of the 'left-end position' and the 'right-end position' does not appear more than once. In order to carry out this processing, for example, the index for the combination of the values in the columns at the left-end position and the right-end position is defined, and by referring the index upon adding a new row to the table, it is determined whether the same combination of the values in the columns at the left-end position and the right-end position exists in the rows, which have been already stored in the table. Here, the index includes the value of combination of the column, which is named the left-end position of the table, and the column, which is named the right-end position of the table, as 'key'; and includes the table number or the value in the column for uniquely specifying the row of the table as 'value'. If the row, which has the same combination of values in the columns at the left-end position and the right-end position as that of the new row to be added, already exists in the table, the addition of the row to be added to the table is cancelled. If the row, which has the same combination of the values in the columns at the left-end position and the right-end position as that of the new row to be added, has not yet been stored in the table, a row is added to the table. Consequently, the set of border base sequences is acquired.

Next, the integration of elements of the set of border base sequences is carried out. This integration is carried out between the base sequences having the same value in column of expressed gene. Therefore, the border base sequences of the exon 1101, 1102, and 1103 are integrated with the border base sequences of the expressed gene formed by the exon 1101, 1102, and 1103, not with the expressed gene formed by the exon 1101, 1102, and 1104. For this purpose, for example, in the table, sorting based on the value in the column of expressed gene is carried out, the table is separated by grouping rows having the same value in the column of expressed gene, and to the respective separated tables, the processing indicated by the flow chart of Fig. 9 is carried out. The reason for this integration between the groups of rows having the same values in the column of expressed gene is to prevent the generation of the base sequence, which never exists in the expressed gene. Consequently, by such processing, the base sequence 1113 and 1114 are acquired.

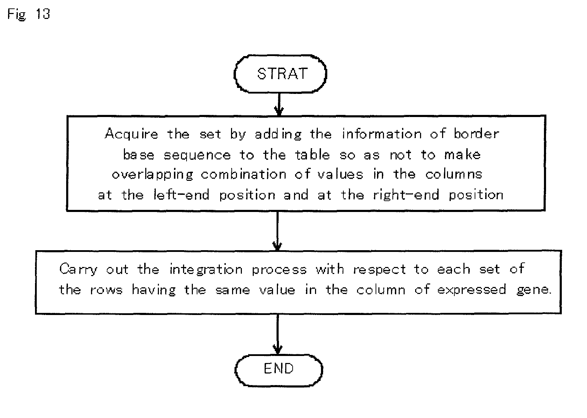

Fig. 13 is a flow chart of the integration process for the set of border base sequences as described above. In the first step, the information of border base sequence is added to the table so as not to make an overlapping combination of values in the columns at the left-end position and at the right-end position. In the next step, the integration process is carried out with respect to each set of the rows having the same value in the column of expressed gene. Therefore, by grouping the table so that the values in columns of expressed gene are the same (e.g. by using 'group by clause' in SQL (Structured Query Language)), the table is separated into some sub tables, and the processing indicated by the flow chart of Fig. 9 is carried out on the respective small tables.

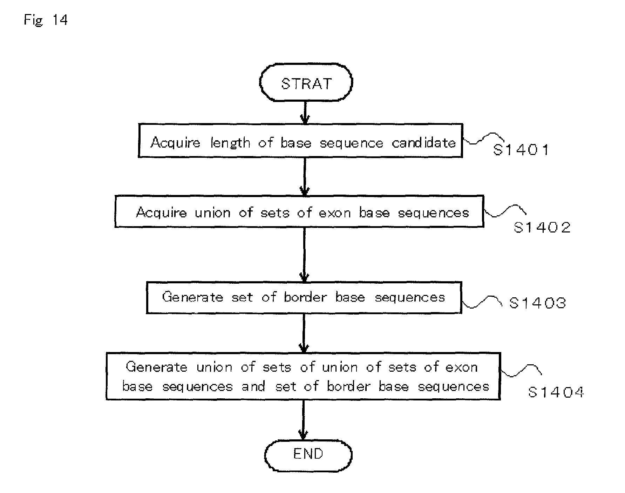

Fig. 14 is a flow chart for the process of the generation method for a set of the base sequences of the first embodiment of the present invention. The generation method for a set of base sequences of the first embodiment comprises an acquisition step for length of base sequence candidate, an acquisition step for set of exon base sequences, a generation step for set of border base sequences, and a generation step for union of sets. Each of these steps corresponds to S 1401, S1402, S1403, and S1404 in the flow chart of Fig. 14, respectively. As described hereinbelow, it is possible to carry out these steps with a computer program. In addition, the above-mentioned program may be recorded on a medium such as a flexible disk, an optical disk, or a memory stick.

The 'acquisition step for length of base sequence candidate' (S1401) is a step, which acquires the length of a specific base sequence candidate (hereinafter, referred to as 'length of base sequence candidate') appearing in a base sequence of an expressed gene. The upper limit of the acquired length of base sequence candidate is preferably less than 30 base sequences, more preferably less than 22, and even more preferably less than 20, and the lower limit thereof is preferably more than 13, more preferably more than 16, and even more preferably more than 18, if the set of base sequences generated by the generation method for a set of base sequences of the first embodiment is used for designing ₛᵢRNA. For example, 19 is the preferable value. In addition, if the set of base sequences is used for designing oligo-DNA of a microarray, the upper limit thereof is preferably less than 30.

The 'acquisition step for set of exon base sequences' (S1402) acquires a union of sets of exon base sequences. In the present specification, the term 'acquisition' includes generation. In cases where the union of sets of exons is generated, it is generated as described in the above fourth section.

The 'generation step for set of border base sequences' (S1403) generates a set of border base sequences. The 'set of border base sequences' is a set of base sequences by integrating information indicating a base sequence, which has the same expressed gene and overlapping position of base sequence, to the set of information, which indicates a base sequence straddling the exon border in the expressed gene formed by a plurality of exons, and indicates the base sequence of the same length as that acquired by the acquisition step for length of base sequence candidate. Specifically, the set of base sequences acquired by the processes described in the fifth section, or the sixth and seventh sections.

The 'generation step for union of sets' (S1404) is a step, which generates a union of sets of the base sequence acquired by the acquisition step for set of exon base sequences, and the set of the base sequences generated by the generation step for set of border base sequences. The union of sets in this step is basically acquired by the operation for acquiring simple sum of sets. However, as exceptions, there are two cases in which the operation for acquiring sum of sets is not simple. At the outset, in cases where the base sequence, which is an element of the union of sets of exon base sequences, is located in the end of expressed gene, and is less than N-1mer, exists, the base sequence is included in the border base sequence or in the base sequence, which is an integration of the border base sequences (therefore, inclusion relation), so that it is necessary to exclude such a base sequence. Moreover, in cases where the base sequence, which is an element of the union of sets of exon base sequences, is located not in the end but in the middle of expressed gene, and is less than 2N-2mer, exists, it is possible that the base sequence is included in the border base sequence or in the base sequence, which is an integration of the border base sequences (in the case of being less than N-1mer, it is certainly included), so that if such a base sequence exists, it is excluded.

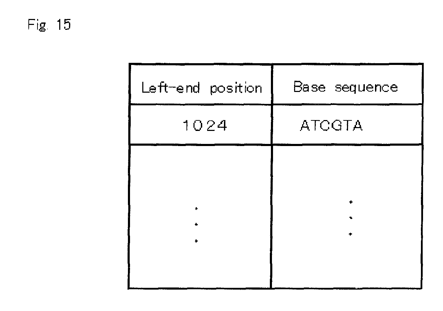

Fig. 15 is a table storing the base sequence acquired by the generation step for union of sets S 1404 of Fig. 14. For example, in the column of 'left-end position', the position of the left-end base of the base sequence in DNA sequence is stored, and in the column of 'base sequence', the base sequence is stored. In addition, the column for storing the information such as the identifier of expressed gene may be generated.

The search is carried out on the set of base sequences generated according to the first embodiment, so that it becomes possible to effectively determine the specific base sequence appearing in the target gene. Consequently, if the base sequence appearing in expressed gene sequence is specific, the number of search results is one, and if not, the number of search results is multiple.

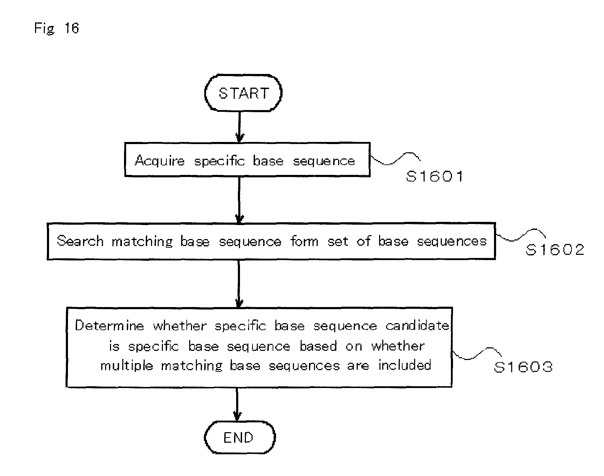

Fig. 16 is a flow chart of the method for searching for a specific base sequence of the second embodiment of the present invention. The method for searching for a specific base sequence of the second embodiment comprises an acquisition step for a specific base sequence candidate, a searching step for a specific base sequence, and a determination step. As described hereinbelow, it is possible to carry out these steps using a computer program. In addition, the above-mentioned computer program may be recorded on a medium such as a flexible disk, an optical disk, or a memory stick.

The 'acquisition step for specific base sequence candidate' (S1601) acquires a specific base sequence candidate. The 'specific base sequence candidate' is a candidate of a specific base sequence appearing in a base sequence of an expressed gene.

Although any base sequence can be a candidate, for example, by the method known as the conventional technology, it is evaluated whether the possibility that the base sequence specifically appears is high, so that the base sequence that was highly evaluated as the specific base sequence may be a candidate. Here, in the method known as the conventional technology: (1) the base sequence, which is identical or similar to the base sequence information of the expressed gene, is searched for from the base sequence information published in the database such as RefSeq of NCBI by using the existing homology search means such as BLAST, FASTA, or ssearch; (2) the summation of the inverse of the value indicating the degree of identity or similarity is computed based on the total amount of the base sequence information of the gene unrelated to the expressed gene among the searched base sequences, or on the value, which indicates the degree of identity or similarity, and is added to the base sequence information of gene unrelated to the expressed gene, such as 'E value' in BLAST, FASTA, or ssearch; and (3) it is determined whether the base sequence specifically appears in the expressed gene based on the above summation, for example, on the amount of the summation. In order to cause a computer to carry out the acquisition step for a specific base sequence candidate, the computer is caused to read the strings indicating the specific base sequence candidate inputted by a keyboard etc.

The 'searching step for specific base sequence' (S 1602) searches for a matching base sequence from a set of base sequences. The 'set of base sequences' includes a union of sets of a union of sets of exon base sequences, and a set of border base sequences. The set of base sequences is, for example, a union of sets of a union of sets of exon base sequences described in the first section, and a set of border base sequences described in the second section, or may be the set generated by the generation method for set of base sequences of the first embodiment. The union of sets of exon base sequences may be acquired by the integration process to the exon base sequence described in the fourth section. In addition, the set of base sequences may further include the sequence, which is uncertain to be an exon or a sequence straddling the border, because of non-decoding of the genome sequence thereof etc. In some cases, the set of base sequences may be the entire set of gene sequences. In addition, as described at the end of the fourth section, to the element of the union of sets of exon base sequences, the information indicating sequence position of exon or the information for identifying the gene formed by the exon may be correlated.

The 'border base sequences' is the same as that described in the second section. Therefore, it is the base sequence, which straddles exon border in the expressed gene formed by a plurality of exons, and has the same length as that of the base sequence of the specific base sequence candidate. The 'matching base sequence' is a base sequence matching a base sequence indicated by the specific base sequence candidate acquired by the acquisition step for a specific base sequence candidate. Here, the term 'two base sequences match with each other' means that the bases forming the two base sequences are compared with respect to each pair, so that the pair not fulfilling a predetermined binomial relation is less than a predetermined number. Here, in many cases, the binomial relation means that the bases forming pairs are identical. Therefore, in terms of mathematical set theory, the binomial relation fulfills only the reflexive law. In addition, the binomial relation, by considering that G and U in the base are easily concatenated, may be used. In addition, it may be determined whether the two base sequences are a match by considering the number of successive matching base sequences, not by depending only on the binomial relation. The term 'less than a predetermined number' means, for example, less than 20%, preferably less than 10%, more preferably less than 5%. As to the above search method, the study is developed in the field of bioinformatics, and the searching method uses a computer such as FASTA, BLAST, and Smith-Waterman dynamic programming algorithm (e.g. 'Bioinformatics:Sequence and Genome Analysis', David W. Mount, Cold Spring Harbor Laboratory Press, 2001 etc.)

The 'determination step' (S 1603) determines whether the specific base sequence candidate acquired by the acquisition step for a specific base sequence candidate is a specific base sequence based on whether a plurality of matching base sequences are included in the search result by the searching step for a specific base sequence. Here, the 'specific base sequence' means the base sequence specifically appearing in the expressed gene. In the determination step, if the matching base sequence is 1 in the search result, it can be determined that the specific base sequence candidate is the specific base sequence. If the matching base sequences are more than 2 in the search result, it is determined that it is not the specific base sequence. If the matching base sequence is 0 in the search result, it is determined that nothing having similarity appears. In cases where the matching base sequence is 0 in the search result, it is inferable that the base sequence candidate has no effect. Therefore, by acquiring the number of sets of the search results, a computer is caused to carry out the determination step.

According to the third embodiment of the present invention, in the method for searching for a specific base sequence according to the second embodiment, the set of border base sequences is the set acquired through integration as described in the fourth and seventh sections.

Therefore, the set of border base sequences is acquired based on a set acquired through integrating information indicating a base sequence, which has the same expressed gene and overlapping position as the base sequence, to the set of information, which indicates (1) a base sequence straddling the exon border in the expressed gene formed by a plurality of exons, and indicates (2) the base sequence of the same length as that of the base sequence of the specific base sequence candidate. Note that it is not necessary to carry out the integration process until the integration becomes impossible, therefore, until the integration is complete. In addition, through the integration, there is the case that the base sequence, which is included in the base sequence acquired through integration, appears in the union of sets of exon base sequence. In this case, as described in the first embodiment, it is necessary to exclude such a base sequence.

The information indicating a base sequence corresponds, for example, to the respective columns stored in the table of Fig. 8, or to the respective columns stored in the table of Fig. 12.

According to the third embodiment, through the integration, it becomes possible to reduce elements to be searched for, thereby downsizing the sets, and improving search speeds.

The fourth embodiment of the present invention is the method for searching for a specific base sequence according to the second or third embodiment comprising an acquisition step for the allowable number of matches.

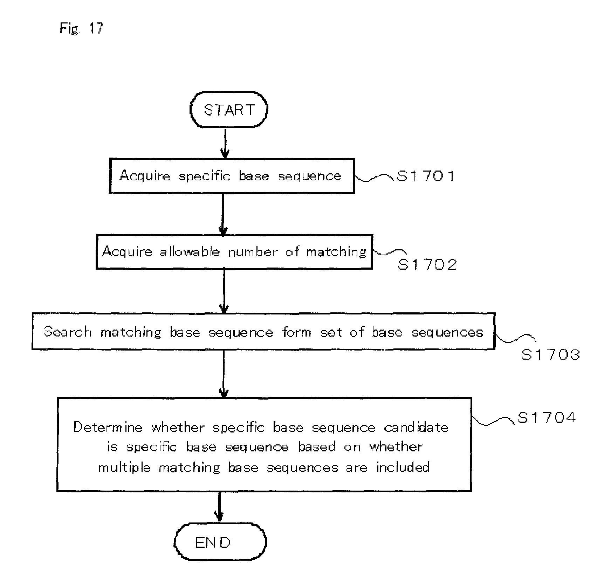

Fig. 17 is a flow chart of the method for searching for a specific base sequence of the fourth embodiment. In this flow chart, the acquisition step for the allowable number of matches S 1702 is added to Fig. 16.

The 'acquisition step for the allowable number of matches' acquires the allowable number of matches. The 'allowable number of matches' is a numerical value, which indicates how many mismatching bases are allowed, as the degree of matching between the base sequence included in the set of base sequences and the base sequence indicated by the specific base sequence candidate. The value is preferably any one of 1, 2, 3, 4, or 5. Here, the 'mismatching of bases' means that the pair of bases does not fulfill a predetermined binomial relation. In order to cause a computer to carry out the acquisition step for the allowable number of matches, for example, the computer is caused to read the allowable number of matches inputted by a keyboard or by selecting a radio button indicated on a screen.

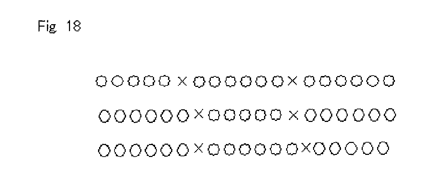

According to the fourth embodiment, in the search step for the base sequence, the search is carried out based on the allowable number of matches acquired by the acquisition step for the allowable number of matches. For example, the search is carried out using the above-mentioned BLAST etc. In this case, the terms 'based on the allowable number of matches' means that the search is carried out so that the number of mismatching base pairs is less than the allowable number of matches. However, since in BLAST, normally, the search is carried out using the portion, in which seven successive bases are the same, in cases where the length of base sequence candidate is 19 and the allowable number of matches is 3, it is impossible to carry out the search for mismatch at the position indicated by '×' in Fig. 18. Accordingly, in the specific base sequence candidate, the base sequence, in which the base at the position indicated by '×' is replaced by the other base, is generated, so that the search for the base sequence, which is identical or complementary to the base sequence indicated by the specific base sequence candidate, may be carried out. Note that an example of the search method by specifying the allowable number of matches includes the method described in 'Computing Highly Specific and Noise-Tolerant Oligomers Efficiently', Tomoyuki YAMADA and Sinichi MORISHITA, to be published in Journal of Bioinformatics and Computational Biology, Imperial College Press.

As the fifth embodiment of the present invention, the method for searching for a specific base sequence, comprising an acquisition step for mismatching base pair, which acquires a base pair, which is determined to be a mismatch by the searching step for base sequence, will be described.

In the method for searching for a specific base sequence of the fifth embodiment, the method for searching for a specific base sequence of the fourth embodiment further comprises an acquisition step for mismatching base pair.

The 'acquisition step for mismatching base pair' acquires a base pair, which is determined to be a mismatch by the searching step for base sequence. This acquisition is carried out by acquiring the base pair inputted by a keyboard connected with a computer, by reading information indicating the base pair recorded on a medium, or by acquiring information inputted via a communication line. In the acquisition step for mismatching base pair, normally, the base, which is not identical, is determined to be mismatching. However, for example, since it is known that G and U are concatenated, thereby forming a pair, there is the case that the pair of G and U is not determined to be mismatching. For this reason, in the fifth embodiment, it is possible to acquire the base pair determined to be mismatching. In addition, instead of acquiring the base pair determined to be mismatching, by acquiring the base pair determined to be matching, the base pair determined to be mismatching may be acquired indirectly. In addition, the base pair to be acquired may be acquired correlated with the degree of matching or mismatching. For example, in the case of the pair of the same bases, the value 1 may be assigned, and in the case of the pair of G and U, the value 0.5 may be assigned. Note that the acquisition step for mismatching base pair is carried out before carrying out the search step for base sequence S 1703. For example, after carrying out the acquisition step for mismatching base pair, the flow chart of Fig. 17 is carried out.

As the sixth embodiment of the present invention, the method for searching for a specific base sequence, in which a distribution of occurrence of a mismatching base is specified, and the search is carried out.

In the method for searching for a specific base sequence of the sixth embodiment, the method for searching for a specific base sequence according to any one of the second to fifth embodiments further comprising an acquisition step for distribution information of mismatching.

The 'acquisition step for distribution information of mismatching' acquires distribution information as degree of matching between the base sequence included in the set of base sequences and the base sequence indicated by the specific base sequence candidate. The 'distribution information' is information indicating a distribution of occurrence of mismatching. Examples of the distribution information include the information indicating that more than two mismatching bases do not appear successively, the information indicating that there are less mismatches at the 5'-end of the specific base sequence, and the information indicating that the number of occurrences of successive mismatches between the specific base sequence and the base is less than a predetermined number of times. The purpose of acquiring the distribution information is that, for example, even if the same number of mismatches of the bases, in cases where the mismatching of the bases occurs successively, it becomes difficult for the nucleic acid to be hybridized, so that the base sequence, in which the mismatch of the bases occurs successively, is excluded, even if the allowable number of matches is fulfilled. In addition, in cases where the bases, which are mismatching but are not determined to be mismatching, since the hybridization can be caused despite the mismatching portion, in order to exclude it, it is specified that the bases, which are not determined to be mismatching, do not successively occur more than the predetermined value.

The distribution information may be, for example, a program for determining whether a distribution of mismatches of bases is a predetermined distribution. Alternatively, it may be the information for selecting some types of distribution of mismatches of bases, which are preliminarily determined. For example, it may be the information indicating the number, which is assigned to the distribution of mismatches of bases.

In the sixth embodiment, the processing of the acquisition step for distribution information of mismatching is carried out as follows. Therefore, the search is carried out in further consideration of the distribution information acquired by the acquisition step for distribution information of mismatching. For example, the search in any one of the second to fifth embodiments is carried out at the outset, thereby selecting the information fulfilling the distribution information of mismatching such as the information indicating that more than two mismatching bases do not appear successively, the information indicating that there are less mismatches at the 5'-end of the specific base sequence, and the information indicating that the number of occurrences of successive mismatches between the specific base sequence and the base is less than a predetermined number of times, from the search result.

The method for searching for a specific base sequence of the seventh embodiment of the present invention is the method for searching for a specific base sequence according to any one of the second to sixth embodiments, wherein the specific base sequence candidate is a candidate of a base sequence of oligo-DNA for microarray.

Thus, it is not necessary to examine the search result as in the conventional technology, thereby carrying out designing oligo-DNA in microarray, effectively.

The method for searching for a specific base sequence of the eighth embodiment of the present invention is the method for searching for a specific base sequence according to any one of the second to sixth embodiments, wherein the specific base sequence candidate is a candidate of a base sequence of ₛᵢRNA.

Thus, it is not necessary to examine the search result as in the conventional technology, thereby carrying out designing ₛᵢRNA, effectively.

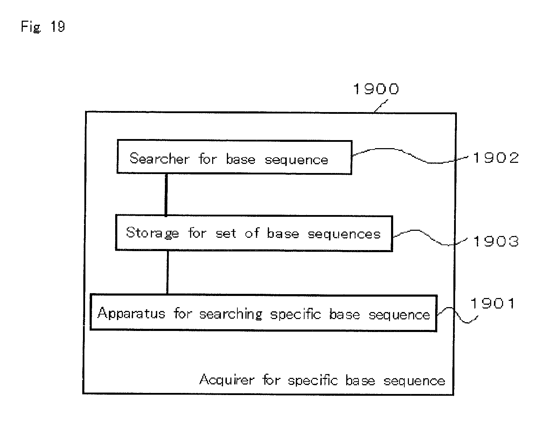

Fig. 19 is the apparatus for searching for a specific base sequence of the ninth embodiment of the present invention. The apparatus for searching for a specific base sequence of the ninth embodiment is an apparatus for using, for example, the method for searching for a specific base sequence of the second embodiment

The apparatus for searching for a specific base sequence 1900 comprises the storage for a set of base sequences 1901, the acquirer for a specific base sequence candidate 1902, and the searcher for a specific base sequence 1903. Note that, in the present specification, the configurations indicated in the functional block diagram are implemented as hardware by a CPU, memory, other LSI of any computer etc. Moreover, they are implemented as software by a program loaded to a memory etc. Furthermore, they may be implemented by a combination of hardware and software. Specifically, in cases where they are implemented by software, these units may be implemented by causing a computer to carry out a program installed thereto. For example, the program is recorded to various recording mediums and is automatically read by a computer to implement the apparatus for searching for a specific base sequence 1900 according to necessity. Here, the 'recording medium' may include any 'transportable type physical medium' such as a flexible disk, an optical disk, a ROM, a EPROM, a EEPROM, a CD-ROM, a MO, a DVD, a flash disk, any 'fixed type physical medium' such as ROM, RAM, or HD mounted in various computer systems, or 'communication medium' for storing the program for a short period such as a communication line or carrier wave in the case of transmitting the program via network typified by LAN, WAN, or Internet. Note that the above computer is not limited to a mainframe computer, and may be an information processing device such as a workstation, or a personal computer. Further, to such an information processing device, peripheral devices such as a printer or a scanner may be connected.

In addition, the 'program' means a data processing method described by any language or description method, and any format such as source code or binary code etc. may be allowed. Note that the 'program' is not necessarily limited to a program having a single configuration, and may include a program having a distributed configuration as multiple modules or library, and a program, which cooperates with other programs typified by operating system, and implements function. Note that, in the apparatus for searching for specific base sequence 1900, general configuration or process may be used for the specific configuration for reading the recording medium, the reading means, or install process after reading etc.

Although not indicated in the drawing, the apparatus for searching for a specific base sequence 1900 may be communicably connected to the external system for providing the external database of information of the base sequence of gene etc. or the external program for homology search etc. via the communication network such as the internet. By this configuration, a website for carrying out the external program. The external system may be configured as a WEB server or ASP server etc. For example, the storage for set of base sequences 1901, and/or the acquirer for specific base sequence candidate 1902 may be communicably connected to the external system. Although the configuration of the communication network is not specifically limited, for example, it is configured by a communication device such as a router, and wired or wireless communication line such as an exclusive line.

The 'storage for set of base sequences 1901' stores the set of base sequences. The 'set of base sequences' is a set, which includes a union of sets of a union of sets of exon base sequences, and a set of border base sequences, which straddles exon borders in the expressed gene formed by a plurality of exons. For example, it is the set generated by the method described in the first embodiment, or the set searched by the searching step for base sequence of the method described in the second embodiment The storage for set of base sequences 1901 stores the set of base sequences as data in a predetermined format in an inputtable/outputtable state by using a memory device such as RAM and ROM, fixed disk drive such as hard disk, or storage device using flexible disk or optical disk. Therefore, in cases where the apparatus for searching for a specific base sequence 1900 is implemented by using a computer, a driver for performing input/output to a device for this storage, and a program module for performing input/output of data by using the driver etc. correspond to the storage for set of base sequences 1901.

The 'acquirer for specific base sequence candidate 1902' acquires a specific base sequence candidate, which is a candidate of a specific base sequence appearing in a base sequence of an expressed gene. For example, the specific base sequence candidate, which is inputted to a text area of a web page indicated in a web browser operated by a computer which communicates via a communication network such as intemet, and is transmitted as text information from the browser by using HTTP (Hypertext Transfer Protocol), is received, thereby acquiring the specific base sequence candidate. Therefore, in cases where the apparatus for searching for a specific base sequence 1900 is implemented by using a computer, communication interface, a driver for performing input/output in the input/output interface for performing input/output of data to a mouse, keyboard, and a display, and a program module for performing input/output of data by using the driver etc. correspond to the acquirer for specific base sequence candidate 1902.

The 'searcher for specific base sequence 1903' searches for a matching base sequence, which is a base sequence matching the specific base sequence candidate acquired by the acquirer for specific base sequence candidate 1902, from the base sequences included in the set of base sequences stored by the storage for set of base sequences. For this search, for example, the program carrying out algorithm (e.g. BLAST), described in any one of the second to fourth embodiments, is used. The search result may be replied to the browser, which transmitted the specific base sequence candidate. For example, the number of the search results may be replied, or the base sequence matching with the specific base sequence candidate may be replied by acquiring the information as to the expressed gene. Further, according to the number of search results, the result of determination as to whether the specific base sequence candidate acquired by the acquirer for specific base sequence candidate 1902 is the specific base sequence may be replied. In addition, it may be determined whether the specific base sequence candidate is the specific base sequence by the program, which is defined by JAVA (registered trademark) etc., operating in the browser. Note that, in cases where the apparatus for searching for specific base sequence 1900 is implemented by using a computer, under the control of the computer's CPU, data passing with the module etc. corresponding to the acquirer for specific base sequence candidate 1902 is carried out, and data passing with the module etc. corresponding to the storage for set of base sequences 1901 is carried out, in addition, the module etc., which carries out the search of the set of base sequences stored in the hard disk etc., corresponds to the searcher for specific base sequence 1903.

In addition, the apparatus for searching for a specific base sequence 1900 may comprise the storage for the search result by the searcher for specific base sequence 1903. In addition, the storage, which correlates the specific base sequence candidate acquired by the acquirer for specific base sequence candidate 1902 with the search result searched by the searcher for specific base sequence 1903, and stores them, may be comprised. By comprising the storage, in cases where the same specific base sequence candidate acquired more than once by the acquirer for specific base sequence candidate 1902, from the second search, the information stored in this storage is searched, thereby improving responsivity.

The tenth embodiment of the present invention is the apparatus for searching for specific base sequence according to the ninth embodiment, wherein the set of border base sequences is acquired based on a set acquired by integrating information indicating a base sequence, which has same expressed gene and overlapping position of base sequence, to the set of information, which indicates a base sequence straddling the exon border in the expressed gene formed by a plurality of exons, and indicates the base sequence of the same length as that of the base sequence of the specific base sequence candidate. The apparatus for searching for specific base sequence of the tenth embodiment is, for example, the apparatus for using the method for searching for specific base sequence of the third embodiment.

Therefore, the apparatus for searching for specific base sequence of the tenth embodiment is the apparatus for searching for specific base sequence, wherein the set of base sequences stored by the storage for set of base sequences 1901 is integrated to the border base sequence, thereby generating the set by integration process described in the seventh section etc.

By the integration, it becomes possible to reduce the number of elements of set of base sequences, thereby saving the disk space used by the storage for set of base sequences 1901, and improving search speed by the reduction of the number of elements.

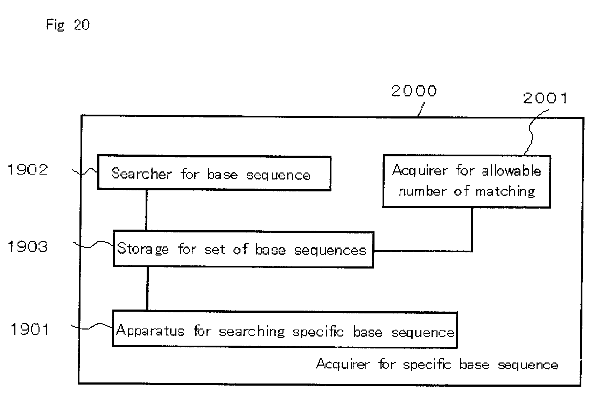

Fig. 20 is a functional block diagram of the apparatus for searching for specific base sequence of the eleventh embodiment of the present invention. The apparatus for searching for specific base sequence 2000 comprises the storage for set of base sequences 1901, the acquirer for specific base sequence candidate 1902, the searcher for specific base sequence 1903, and the acquirer for allowable number of matches 2001. Therefore, the apparatus for searching for specific base sequence of the eleventh embodiment has the configuration, wherein the apparatus for searching for specific base sequence according to the ninth or tenth embodiment comprises the acquirer for allowable number of matches. Note that, in the present specification, the same numbers are assigned to the sections defined as the same. However, in the actual manufacturing, the sections of the same numbers do not have the same configurations, even if they have the same number. The apparatus for searching for specific base sequence of the twentieth embodiment is, for example, the apparatus for using the method for searching for specific base sequence of the fourth embodiment.

The 'acquirer for allowable number of matches 2001' acquires a numerical value, which indicates how many mismatching bases are allowed, as degree of matching between the base sequence included in the set of base sequences and the base sequence indicated by the specific base sequence candidate. For example, when the specific base sequence candidate is transmitted from the browser, the allowable number of matches may be transmitted from the browser. Thus, the acquirer for allowable number of matches 2001 acquires the transmitted allowable number of matches. Further, the configuration, in which the allowable number of matches is directly inputted, may be allowed.

In the eleventh embodiment, the searcher for specific base sequence 1903 carries out search based on the allowable number of matches acquired by the acquirer for allowable number of matches 2001. This method for search is the same as that of the fourth embodiment.

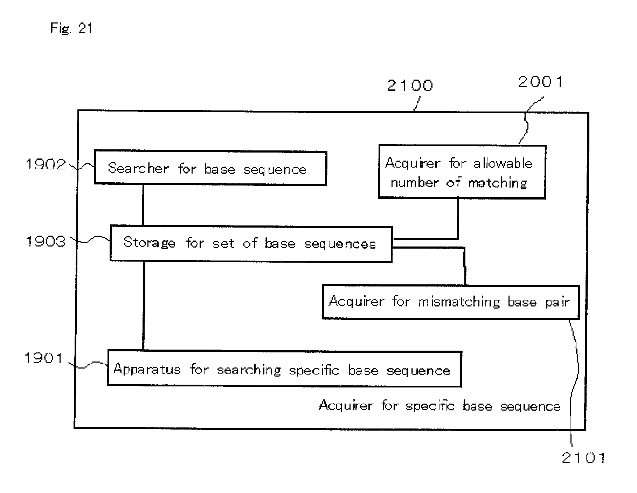

Fig. 21 is a functional block diagram of the apparatus for searching for specific base sequence of the twelfth embodiment of the present invention. The apparatus for searching for specific base sequence 2100 comprises the storage for set of base sequences 1901, the acquirer for specific base sequence candidate 1902, the searcher for specific base sequence 1903, the acquirer for allowable number of matches 2001, and the acquirer for mismatching base pair 2101. Therefore, the apparatus for searching for specific base sequence of the twelfth embodiment has the configuration, wherein the apparatus for searching for specific base sequence according to the eleventh embodiment comprises the acquirer for mismatching base pair 2101. The apparatus for searching for specific base sequence of the twelfth embodiment is, for example, the apparatus for using the method for searching for specific base sequence of the fifth embodiment.

The 'acquirer for mismatching base pair' 2101 acquires a base pair, which is determined to be mismatching by the searcher for base sequence. For example, it acquires text information indicating the base pair, which is determined to be mismatching. Alternatively, by acquiring the base pair, which is determined to be matching (e.g. G and U), the base pair, which is determined to be mismatching, may be acquired indirectly. Therefore, a communication interface, a driver for performing input/output in the input/output interface for performing input/output of data to a mouse, keyboard, and a display, and a program module for performing input/output of data by using the driver etc. correspond to the acquirer for mismatching base pair 2101.

The processing flow of the apparatus for searching for specific base sequence of the twelfth embodiment is the same as that of the apparatus for searching for specific base sequence of the eleventh embodiment. However, before searching for the matching base sequence, the base pair, which is determined to be mismatching by the searcher for base sequence, is acquired by the acquirer for mismatching base pair 2101.

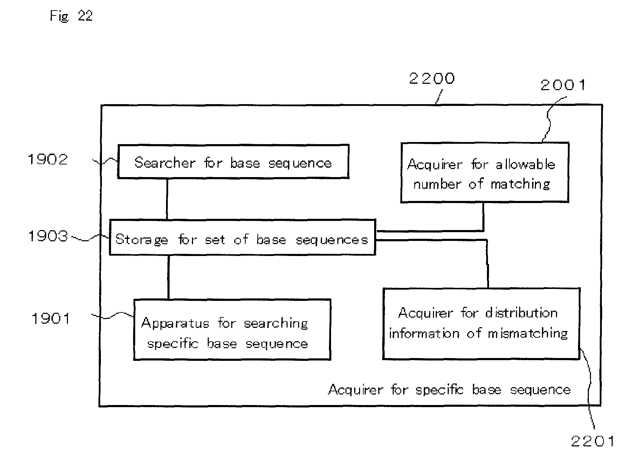

Fig. 22 is a functional block diagram of the apparatus for searching for specific base sequence of the thirteenth embodiment of the present invention. The apparatus for searching for specific base sequence 2200 comprises the storage for set of base sequences 1901, the acquirer for specific base sequence candidate 1902, the searcher for specific base sequence 1903, the acquirer for allowable number of matches 2001, and the acquirer for distribution information of mismatching 2201. In addition, the apparatus for searching for specific base sequence 2200 may further comprise the acquirer for mismatching base pair. Therefore, the apparatus for searching for specific base sequence of the thirteenth embodiment has the configuration, wherein the apparatus for searching for specific base sequence according to any one of the ninth to twelfth embodiment comprises the acquirer for distribution information of mismatching 2201. The apparatus for searching for specific base sequence of the thirteenth embodiment is, for example, the apparatus for using the method for searching for specific base sequence of the sixth embodiment.

The 'acquirer for distribution information of mismatching' 2201 acquires distribution information indicating a distribution of occurrence of mismatching base as degree of matching between the base sequence of the set of base sequence and the base sequence of the specific base sequence candidate. Examples of the distribution information are the same as those of the sixth embodiment. Therefore, a communication interface, a driver for performing input/output in the input/output interface for performing input/output of data to a mouse, keyboard, and a display, and a program module for performing input/output of data by using the driver etc. correspond to the acquirer for distribution information of mismatching 2201.

In the thirteenth embodiment, the searcher for specific base sequence 1903 carries out search based on the distribution information acquired by the acquirer for distribution information of mismatching 2201. For example, the search is carried out as described in the eleventh or twelfth embodiment, and from the intermediate search result, which is the result of that search, the search is carried out based on the distribution information. Therefore, from the intermediate search result, the final search result, which corresponds to the distribution information, is selected.

The fourteenth embodiment of the present invention is the apparatus for storing set of base sequences. Therefore, the apparatus for storing set of base sequences, which stores a set of base sequences including a union of sets of exon base sequences, and a set of border base sequences straddling exon border in the expressed gene formed by a plurality of exons, in a searchable state.

Therefore, for example, the apparatus for storing set of base sequences of the fourteenth embodiment has a configuration, in which a hard disk for implementing the storage for set of base sequences 1901 of the apparatus for searching for specific base sequence 1900 of the eighth embodiment is an external hard disk device. Alternatively, it may be a server comprising a hard disk for implementing the storage for set of base sequences 1901 of the apparatus for searching for specific base sequence 1900.

According to the apparatus for storing set of base sequences of the fourteenth embodiment, it becomes possible to implement searches based on various search algorithms.

The fifteenth embodiment of the present invention is the storage for set of base sequence according to the fourteenth embodiment, wherein the set of border base sequences is acquired based on a set acquired by integrating information indicating a base sequence, which has same expressed gene and overlapping position of base sequence, to the set of information, which indicates a base sequence straddling the exon border in the expressed gene formed by a plurality of exons, and indicates the base sequence of the same length as that of the base sequence as an input for searching. Therefore, the fifteenth embodiment has the configuration, in which the storage for set of base sequences of the apparatus for searching for specific base sequence of the tenth embodiment is the other apparatus. For example, the configuration can be acquired by that the data stored by the storage for set of base sequences of the apparatus for searching for specific base sequence of the tenth embodiment is stored by NAS (Network Attached Storage) or SAN (Storage Area Network).

According to the fifteenth embodiment, the integration process is carried out for the border base sequence, thereby reducing the necessary disk space.

### Industrial Applicability

According to the present invention, the set of base sequences is generated from the exon base sequence and the base sequence appearing in the exon border, and search is carried out, so that it becomes possible to determine whether the base sequence is the specific base sequence appearing in the expressed gene based on the number of the search results. This is effective in determining the specific base sequence.

## Claims

1. A method for searching for a specific base sequence, comprising:
an acquisition step for a specific base sequence candidate, which acquires a specific base sequence candidate, which is a candidate of a specific base sequence appearing in a base sequence of an expressed gene;
a searching step for a specific base sequence, which searches a matching base sequence, which is a base sequence matching the specific base sequence candidate acquired by said acquisition step for specific base sequence candidate, from a set of base sequences, which include a union of sets of
a union of sets of exon base sequences, and
a set of border base sequences, which straddle exon borders in the expressed gene formed by a plurality of exons; and
a determination step, which determines whether the specific base sequence candidate acquired by said acquisition step for a specific base sequence candidate is a specific base sequence based on whether a plurality of matching base sequences are included in the search result by said search step for a specific base sequence.

2. The method for searching for a specific base sequence according to Claim 1, wherein
attribute information including information indicating the position of exon sequence, or information for identifying gene formed by exon, is correlated to an element of said union of set of exon base sequences.

3. The method for searching for a specific base sequence according to Claim 1 or 2, wherein
said set of border base sequences is acquired based on a set acquired by integrating information indicating a base sequence, which has same expressed gene and overlapping position of base sequence, to the set of information, which indicates a base sequence straddling the exon border in the expressed gene formed by a plurality of exons, and indicates the base sequence of the same length as that of the base sequence of said specific base sequence candidate.

4. The method for searching for a specific base sequence according to any one of Claims 1 to 3, comprising:
an acquisition step for allowable number of matches, which acquires a numerical value, indicating the number of allowable mismatching bases, as a degree of matching between the base sequence included in said set of base sequences and the base sequence indicated by said specific base sequence candidate, wherein
said searching step for base sequence carries out search based on the allowable number of matches acquired by said acquisition step for allowable number of matches.

5. The method for searching for a specific base sequence according to Claim 4, comprising:
an acquisition step for mismatching base pair, which acquires a base pair, which is determined to be mismatching by said searching step for base sequence.

6. The method for searching for a specific base sequence according to any one of Claims 1 to 5, comprising:
an acquisition step for distribution information of mismatching, which acquires distribution information indicating a distribution of occurrence of mismatching bases as a degree of matching between the base sequence included in said set of base sequences and the base sequence indicated by said specific base sequence candidate, wherein
said searching step for base sequence carries out search based on the distribution information acquired by said acquisition step for distribution information of mismatching..

7. The method for searching for specific base sequence according to Claim 6, wherein
said distribution information indicates length of successive bases, which are not determined to be mismatching.

8. The method for searching for specific base sequence according to any one of Claims 1 to 7, wherein
said specific base sequence candidate is a candidate of a base sequence of oligo-DNA for microarray.

9. The method for searching for a specific base sequence according to any one of Claims 1 to 7, wherein
said specific base sequence candidate is a candidate of base sequence of _{Si}RNA.

10. An apparatus for searching for a specific base sequence, comprising:
a storage for set of base sequences, which stores a set of base sequences, which includes a union of sets of
a union of sets of exon base sequences, and
a set of border base sequences, which straddles exon border in the expressed gene formed by a plurality of exons;
an acquirer for specific base sequence candidate, which acquires a specific base sequence candidate, which is a candidate of a specific base sequence appearing in a base sequence of an expressed gene; and
a searcher for specific base sequence, which searches for a matching base sequence, which is a base sequence matching the specific base sequence candidate acquired by said acquirer for specific base sequence candidate, from the base sequences included in the set of base sequences stored by said storage for set of base sequences.

11. The apparatus for searching for specific base sequence according to Claim 10, wherein
attribute information, including information indicating position of exon sequence, or information for identifying gene formed by exon, is correlated with an element of said union of sets of exon base sequences.

12. The apparatus for searching for a specific base sequence according to Claim 10 or 11, wherein
said set of border base sequences is acquired based on a set acquired by integrating information indicating a base sequence, which has the same expressed gene and overlapping position of base sequence, as the set of information, which indicates a base sequence straddling the exon border in the expressed gene formed by a plurality of exons, and indicates the base sequence of the same length as that of the base sequence of said specific base sequence candidate.

13. The apparatus for searching for specific base sequence according to any one of Claims 10 to 12, comprising:
an acquirer for allowable number of matches, which acquires a numerical value, indicating the number of allowable mismatching bases, as a degree of matching between the base sequence included in said set of base sequences and the base sequence indicated by said specific base sequence candidate, wherein
said searcher for base sequence carries out search based on the allowable number of matches acquired by said acquirer for allowable number of matches.

14. The apparatus for searching for a specific base sequence according to Claim 13, comprising:
an acquirer for mismatching base pair, which acquires a base pair, which is determined to be mismatching by said searcher for base sequence.

15. The apparatus for searching for a specific base sequence according to any one of Claims 10 to 14, comprising:
an acquirer for distribution information of mismatching, which acquires distribution information indicating a distribution of occurrence of mismatching bases as degree of matching between the base sequence of said set of base sequence and the base sequence of said specific base sequence candidate, wherein
said searcher for base sequence carries out search based on the distribution information acquired by said acquirer for distribution information of mismatching.

16. The apparatus for searching for a specific base sequence according to Claim 15, wherein
said distribution information indicates length of successive bases, which are not determined to be mismatching.

17. An apparatus for storing set of base sequences, storing
a set of base sequences including
a union of sets of exon base sequences, and
a set of border base sequences straddling exon border in the expressed gene formed by a plurality of exons, in a searchable state.

18. The apparatus for storing a set of base sequences according to Claim 17, wherein
attribute information, including information indicating position of exon sequence, or information for identifying gene formed by exon, is correlated to an element of said union of sets of exon base sequences.

19. The storage for set of base sequence according to Claim 17 or 18, wherein
said set of border base sequences is acquired based on a set acquired by integrating information indicating a base sequence, which has the same expressed gene and overlapping position of base sequence, to the set of information, which indicates a base sequence straddling the exon border in the expressed gene formed by a plurality of exons, and indicates the base sequence of the same length as that of the base sequence as an input for searching.

20. A generation method for set of base sequence, comprising:
an acquisition step for length of base sequence candidate, which acquires length of specific base sequence candidate appearing in a base sequence of an expressed gene;
an acquisition step for set of exon base sequences, which acquires a union of sets of exon base sequences;
a generation step for set of border base sequences, which generates a set of base sequences by integrating information indicating a base sequence, which has the same expressed gene and overlapping position of base sequence, to the set of information, which indicates a base sequence straddling the exon border in the expressed gene formed by a plurality of exons, and indicates the base sequence of the same length as that acquired by said acquisition step for length of base sequence candidate; and
a generation step for union of sets, which generates a union of sets of the base sequences acquired by said acquisition step for set of exon base sequences, and set of the base sequences generated by said generation step for set of border base sequences.

21. A searching program for specific base sequence, causing a computer to carry out:
an acquisition step for specific base sequence candidate, which acquires a specific base sequence candidate, which is a candidate of a specific base sequence appearing in a base sequence of an expressed gene; and
a search step for a specific base sequence, which searches for a matching base sequence, which is a base sequence matching a base sequence indicated by the specific base sequence candidate acquired by said acquisition step for a specific base sequence candidate, from a set of base sequences, which includes a union of sets of
a union of sets of exon base sequences, and
a set of border base sequences, which straddles exon borders in the expressed gene formed by a plurality of exons.

22. A generation program for a specific base sequence, causing a computer to carry out:
an acquisition step for length of base sequence candidate, which acquires length of specific base sequence candidate appearing in a base sequence of an expressed gene;
an acquisition step for set of exon base sequences, which acquires a union of sets of exon base sequences;
a generation step for set of border base sequences, which generates a set of base sequence by integrating information indicating a base sequence, which has same expressed gene and overlapping position of base sequence, to the set of information, which indicates a base sequence straddling the exon border in the expressed gene formed by a plurality of exons, and indicates the base sequence of the same length as that acquired by said acquisition step for length of base sequence candidate; and
a generation step for union of sets, which generates a union of set of the base sequences acquired by said acquisition step for set of exon base sequences, and set of the base sequences generated by said generation step for set of border base sequences

23. A search program for a specific base sequence, causing a computer to carry out:
an acquisition step for a specific base sequence candidate, which acquires a specific base sequence candidate, which is a candidate of a specific base sequence appearing in a base sequence of an expressed gene;
a search step for a specific base sequence, which searches for a matching base sequence, which is a base sequence matching a base sequence indicated by the specific base sequence candidate acquired by said acquisition step for specific base sequence candidate, from a set of base sequences, which includes a union of sets of
a union of sets of exon base sequences, and
a set of border base sequences, which straddles exon border in the expressed gene formed by a plurality of exons; and
a determination step, which determines whether the specific base sequence candidate acquired by said acquisition step for specific base sequence candidate is a specific base sequence based on whether a plurality of matching base sequences are included in the search result by said searching step for specific base sequence.
